# EUROPÄISCHE PATENTSCHRIFT

(11) **EP 1 716 109 B1**
(45) Veröffentlichungstag und Bekanntmachung des Hinweises auf die Patenterteilung: **22.09.2010**
(21) Anmeldenummer: 05715209.2
(22) Anmeldetag: 26.01.2005
(51) Int. Cl.: C07C 253/34, C07C 255/04, C07C 255/07

(54) **VERFAHREN ZUR HYDROCYANIERUNG**
METHOD FOR HYDROCYANATION
PROCEDE D'HYDROCYANATION

(30) Priorität: 29.01.2004 DE 102004004718
(43) Veröffentlichungstag der Anmeldung: 02.11.2006
(73) Patentinhaber: BASF SE, 67056 Ludwigshafen (DE)
(72) Erfinder: JUNGKAMP, Tim, B-2950 Kapellen (BE); POLKA, Hans-Martin, 69469 Weinheim (DE); BAUMANN, Robert, 68159 Mannheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE)
(86) Internationale Anmeldenummer: PCT/EP2005/000724
(87) Internationale Veröffentlichungsnummer: WO 2005/073178

(56) Entgegenhaltungen:
- DE-A1- 2 450 863
- US-B1- 6 169 198

## Beschreibung

Die vorliegende Erfindung betrifft ein Verfahren zur Abtrennung von Cyanwasserstoff aus Pentennitril und Cyanwasserstoff enthaltenden Mischungen.

Adipodinitril, ein wichtiges Intermediat in der Nylonproduktion, wird durch zweifache Hydrocyanierung von 1,3-Butadien hergestellt. Dabei wird in einer ersten Hydrocyanierung 1,3-Butadien mit Cyanwasserstoff in Gegenwart von Nickel(0), das mit Phosphorliganden stabilisiert ist, zu 3-Pentennitril umgesetzt. Nebenkomponenten dieser ersten Hydrocyanierung sind im Wesentlichen 2-Methyl-3-butennitril, 2-Pentennitrile, 2-Methyl-2-butennitrile, C₉-Nitrile, Methylglutardinitril und 4-Vinylcyclohexen. In einer zweiten Hydrocyanierung wird anschließend 3-Pentennitril mit Cyanwasserstoff zu Adipodintril ebenfalls an einem Nickel-Katalysator, allerdings unter Zusatz einer Lewis-Säure umgesetzt.

Bei der ersten Hydrocyanierung wird 1,3-Butadien in der Hydrocyanierungsreaktion im Verhältnis zum Cyanwasserstoff im stöichiometrischen Überschuss eingesetzt. Bei der Hydrocyanierung reagiert der eingesetzte Cyanwasserstoff nahezu vollständig ab. Allerdings verbleibt im Reaktionsaustrag aus dieser Hydrocyanierung im Allgemeinen ein Restgehalt an Cyanwasserstoff von bis zu 5000 Gew.-ppm. Dieser Restgehalt an Cyanwasserstoff führt in den nachfolgenden Verfahrensstufen, in denen der Hydrocyanierungskatalysator vom Reaktionsprodukt der Pentennitril-Isomere abgetrennt wird, durch Erhitzen zu Fouling und Bildung von Feststoffen, die Nickel(II)-cyanid enthalten.

In den Hydrocyanierungsausträgen liegt Cyanwasserstoff nicht nur als physikalisch gelöste. Komponente vor, sondern auch als chemisch am Nickelkomplex gebundene Spezies. Deswegen ist der beobachtete Dampfdruck des Cyanwasserstoffs im Reaktionsaustrag häufig niedriger als der aus dem Molenbruch und dem Dampfdruck des Cyanwasserstoffes nach dem Raoult'schen Gesetz berechnete. Durch diese Dampfdruckreduktion ist die Abtrennung von Cyanwasserstoff aus Pentennitril enthaltenden Mischungen durch Destillation erschwert.

Um in der betrieblichen Praxis Reste von Cyanwasserstoff aus Hydrocyanierungsausträgen durch Destillation vollständig abzutrennen, werden im Allgemeinen folgende Wege vorgeschlagen, wobei eine vollständige Entfernung von Cyanwasserstoff bedeutet, dass der Hydrocyanierungsaustrag nach Entfernen des Cyanwasserstoffs noch einen Restgehalt von 0 bis 500 Gew.-ppm Cyanwasserstoff aufweist. So werden entweder sehr geringe Drücke und moderate Temperaturen von 50 bis 70 °C im Sumpf einer geeigneten Destillationsvorrichtung verwendet, um durch Destillation Cyanwasserstoff vollständig zu entfernen. Dabei geht unter Umständen aber auch Wertprodukt in Form von Isomeren des Pentennitrils über. Niedrige Drücke erfordern darüber hinaus tiefe Kondensationstemperaturen für das abdestillierte Material, das aus wirtschaftlichen Gründen in den Prozess zurückgeführt werden muss. Die Anwendung von hohen Temperaturen, beispielsweise 70 bis 120 °C, ist technisch realisierbar, führt aber gleichzeitig dazu, dass Reste an Cyanwasserstoff mit dem Nickelkatalysator zu Feststoffen, die Nickel(II)-cyanid enthalten, reagieren und somit ein Fouling in den Verdampferstufen mit sich bringen.

DE-2450863 offenbart eine derartige Destillation.

Die Aufgabe der vorliegenden Erfindung ist demgemäß ein Verfahren zur Verringerung des Gehaltes von Cyanwasserstoff in Pentennitril und Cyanwasserstoff enthaltenden Mischungen bereitzustellen, das die zuvor beschriebenen Nachteile vermeidet.

Die Lösung dieser Aufgabe geht aus von einem Verfahren zur Verringerung des Gehaltes von Cyanwasserstoff in Pentennitrilen enthaltenden Mischungen. Das erfindungsgemäße Verfahren ist dann dadurch gekennzeichnet, dass die Verringerung des Gehaltes an Cyanwasserstoff durch eine Abtrennung von Cyanwasserstoff mittels einer azeotropen Destillation mit 1,3-Butadien erfolgt.

Das erfindungsgemäße Prinzip beruht auf dem Effekt, dass Cyanwasserstoff beim Verdampfen von 1,3-Butadien aus einem Reaktionsaustrag von Hydrocyanierungen in die Dampfphase gelangt. Erfindungsgemäß wurde dabei gefunden, dass selbst bei einer fraktionierten Destillation von 1,3-Butadien aus Gemischen, die 1,3-Butadien (Sdp.^{1013 mbar} = -4 °C) und Cyanwasserstoff (Sdp.^{1013 mbar} = +27 °C) enthalten, trotz des großen Siedepunktunterschieds von 31 °C, immer Cyanwasserstoff im Kopfaustrag vorliegt. Cyanwasserstoff und 1,3-Butadien bilden ein Siedepunktminimumazeotrop, so dass unabhängig von den Bedingungen, unter denen der Hydrocyanierungsaustrag teilverdampft wird, immer Cyanwasserstoff in Mischungen mit 1,3-Butadien über Kopf geht.

In einer besonders bevorzugten Ausführungsform ist das erfindungsgemäße Verfahren durch die folgenden Verfahrensschritte gekennzeichnet:
(1) Hydrocyanierung von 1,3-Butadien durch dessen Umsetzung mit Cyanwasserstoff in Gegenwart mindestens eines Hydrocyanierungskatalysators unter Erhalt eines Hydrocyanierungsstromes, der 3-Pentennitril, 2-Methyl-3-butennitril, Cyanwasserstoff, 1,3-Butadien und den mindestens einen Hydrocyanierungskatalysator enthält,
(2) Abtrennung eines Gemisches aus Cyanwasserstoff und 1,3-Butadien, das ein Azeotrop bildet, aus dem Hydrocyanierungsstrom durch Destillation.

Die Hydrocyanierung von 1,3-Butadien zu 3-Pentennitril wird in Gegenwart eines Nickel(0)-Katalysator-Komplexes durchgeführt.

Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel lauf:

P(X¹R¹)(X²R²)(X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I sein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel Ia

(o-Tolyl-O-)_{w}(m-Tolyl-O-)ₓ(p-Tolyl-O-)_{y}(Phenyl-O-)_{z}P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P(O-R¹)ₓ(O-R²)_{y}(O-R³)_{z}(O-R⁴)ₚ (Ib)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

| | | | |
|---|---|---|---|
| Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist. | | | |

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 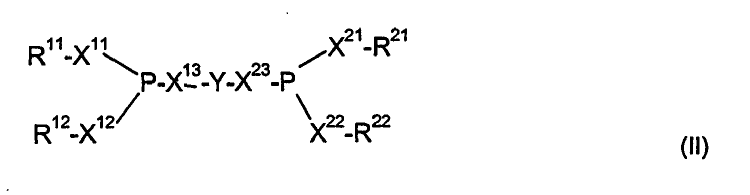 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- γ: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P(O-R¹)ₓ(O-R²)_{y}(C-R³)_{z}(O-R⁴)ₚ (Ib)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Die Hydrocyanierung kann in jeder geeigneten, dem Fachmann bekannten Vorrichtung durchgeführt werden. Für die Reaktion kommen übliche Vorrichtungen in Betracht, wie sie beispielsweise in: Kirk-Othmer, Encyclopedia of Chemical Technology, 4. Ed. Vol. 20, John Wiley & Sons, New York 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Vorrichtungen durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Batchfahrweise, kontinuierlich oder im Semibatchbetrieb durchgeführt werden.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn die Hydrocyanierung im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Hydrocyanierungsreaktion kann durchgeführt werden, indem die Vorrichtung mit allen Reaktanten bestückt wird. Bevorzugt ist allerdings, wenn die Vorrichtung mit dem mindestens einen Katalysator, 1,3-Butadien und gegebenenfalls dem Lösemittel gefüllt wird. Der gasförmige Cyanwasserstoff schwebt vorzugsweise über der Oberfläche der Reaktionsmischung oder wird vorzugsweise durch die Reaktionsmischung geleitet. Eine weitere Verfahrensweise zum Bestücken der Vorrichtung ist das Befüllen der Vorrichtung mit dem mindestens einen Katalysator, Cyanwasserstoff und gegebenenfalls dem Lösemittel und langsames Zuspeisen des 1,3-Butadiens zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Die Hydrocyanierung wird vorzugsweise bei Drücken von 0,1 bis 500 MPa, besonders bevorzugt 0,5 bis 50 MPa, insbesondere 1 bis 5 MPa durchgeführt. Die Reaktion wird vorzugsweise bei Temperaturen von 273 bis 473 K, besonders bevorzugt 313 bis 423 K, insbesondere bei 333 bis 393 K durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Hydrocyanierung kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Hydrocyanierung kann in einer weiteren Ausführungsform in flüssiger Phase durchgeführt werden, wobei der Druck im Reaktor so bemessen ist, dass alle Edukte wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

In dem Verfahrensschritt (2) wird ein ein Azeotrop bildendes Gemisch aus Cyanwasserstoff und 1,3-Butadien aus dem in Verfahrensschritt (1) erhaltenen Hydrocyanierungsstrom durch Destillation abgetrennt.

Das hierfür in Verfahrensschritt (2) verwendete 1,3-Butadien stammt vorzugsweise aus dem Verfahrensschritt (1). Alternativ ist es auch möglich, dass das in Verfahrensschritt (2) verwendete 1,3-Butadien nicht aus dem Hydrocyanierungsschritt (1) stammt und dem Hydrocyanierungsstrom, der Verfahrensschritt (1) verlässt, vor der azeotropen Destillation zugesetzt wird. In einer weiteren Ausführungsform des erfindungsgemäßen Verfahrens ist es möglich, dass das in Verfahrensschritt (2) verwendete 1,3-Butadien zum Teil aus dem Verfahrensschritt (1) stammt und zum anderen Teil dem Hydrocyanierungsstrom, der den Verfahrensschritt (1) verlässt, vor der azeotropen Destillation zugesetzt wird.

Eine weitere Ausführungsform sieht vor, das gesamte 1,3-Butadien, das dem Verfahrensschritt (1) zugeführt wird, dem Strom, der aus dem Verfahrensschritt (1) zum Verfahrensschritt (2) geführt wird, zuzusetzen und von Verfahrensschritt (2) in Verfahrensschritt (1) zurückzuführen.

Das zu Verfahrensschritt (2) zugeführte 1,3-Butadien wird vorzugsweise durch Vorbehandlung an Stabilisator und/oder Wasser abgereichert, wie in der DE-A-102 004 004 684 beschrieben. Im Allgemeinen erfolgt die Abreicherung an Stabilisator und/oder Wasser durch Inkontaktbringen des 1,3-Butadiens mit mindestens einem mikroporösen Feststoff, wobei der mikroporöse Feststoff vorzugsweise ausgewählt ist aus der Gruppe, bestehend aus Aluminiumoxiden und Molsieben und eine Porengröße von 0,01 bis 20 mm aufweist.

Das auf die Masse bezogene Verhältnis von 1,3-Butadien zu Cyanwasserstoff beträgt im Verfahrensschritt (2) vorzugsweise 1 bis 2000, besonders bevorzugt 2 bis 100, insbesondere 5 bis 40.

Der in Verfahrensschritt (1) erhaltene Hydrocyanierungsstrom bzw. die Pentennitril enthaltende Mischung weist vorzugsweise mindestens einen der folgenden Gehalte auf:
- 10 bis 99 Gew.-%, besonders bevorzugt 15 bis 95 Gew.-%, insbesondere 20 bis 90 Gew.-% Pentennitrile, umfassend trans-3-Pentennitril, 2-Methyl-3-butennitril sowie weitere Pentennitrilisomere, wobei je nach verwendetem Katalysatorsystem und Reaktionsbedingungen das Verhältnis von 2-Methyl-3-butennitril zu trans-3-Pentennitril 0,1 : 1 bis 5 : 1 betragen kann;
- 0 bis 60 Gew.-%, besonders bevorzugt 1 bis 30 Gew.-%, insbesondere 5 bis 25 Gew.-%, 1,3-Butadien;
- 0,01 bis 30 Gew.-%, besonders bevorzugt 0,01 bis 20 Gew.-%, insbesondere 0,01 bis 10 Gew.-%, Katalysatorkomponenten einschließlich Katalysatorabbauprodukte,
- besonders bevorzugt 1 Gew.-ppm bis 5 Gew.-%, insbesondere 5 bis 5000 Gew.-ppm, Cyanwasserstoff.

Die azeotrope Destillation von 1,3-Butadien und Cyanwasserstoff in Verfahrensschritt (2) erfolgt vorzugsweise bei einer Temperatur im Sumpf einer geeigneten Destillationsvorrichtung von 30 bis 250 °C, besonders bevorzugt 40 bis 150 °C, insbesondere 50 bis 120 °C, und bei einer Temperatur bei der Kondensation in einer geeigneten Destillationsvorrichtung von -50 bis 150 °C, besonders bevorzugt -15 bis 60 °C, insbesondere -5 bis 45 °C. Dabei beträgt der Druck vorzugsweise 0,001 bis 100 bar, besonders bevorzugt 0,01 bis 10 bar, insbesondere 0,02 bis 5 bar.

Verfahrensschritt (2) kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seiten 334 - 348 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung ausgerüstet, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet.

Die Destillation kann man in mehreren, wie zwei oder drei Vorrichtungen, vorzugsweise in zwei Vorrichtungen, durchführen.

Eine entsprechende zweistufige Destillation als besonders bevorzugte Ausführungsform ist in der DE-A-102 004 004 724 beschrieben, dessen diesbezügliche Offenbarung durch Bezugnahme in die vorliegende Erfindung eingeschlossen ist.

Demnach kann Verfahrensschritt (2) zweistufig in einem Verfahrensschritt (2a) und einem Verfahrensschritt (2b) durchgeführt werden.

Der Verfahrensschritt (2a) des erfindungsgemäßen Verfahrens kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungsko-Ionnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann zudem einstufig im Sinne einer Teilverdampfung des Zulaufstroms erfolgen.

In einer bevorzugten Ausführungsform des erfindungsgemäßen Verfahrens sind in der Destillationsvorrichtung Kolonneneinbauten mit strukturierter Packung vorhanden, die vorzugsweise zwischen 2 und 60, besonders bevorzugt zwischen 3 und 40, insbesondere zwischen 4 und 20, Trennstufen erzeugen.

In einer besonders bevorzugten Ausführungsform des erfindungsgemäßen Verfahrens wird die mindestens eine zur Destillationsvorrichtung von Verfahrensschritt (2a) gehörige Verdampferstufe so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer durch kurze Kontaktzeiten des Materials an der Verdampferoberfläche und möglichst geringe Temperaturen der Verdampferoberflächen erreicht wird.

In einer bevorzugten Ausführungsform des erfindungsgemäßen Verfahrens wird die Destillationsvorrichtung von Verfahrensschritt (2a) so wie in der DE-A-102 004 004 724 beschrieben mit einem geteilten Sumpf betrieben.

Die Destillation kann in einer weiteren bevorzugten Ausführungsform des erfindungsgemäßen Verfahrens mit einem Direktkondensator ausgeführt werden, so dass die Kondensation in einem Kolonnenschuss durchgeführt wird, der vorzugsweise ausgestattet ist mit einer strukturierten Kolonnenpackung, einer Fangtasse unterhalb dieser Packung, einem flüssigen Abzug aus der Fangtasse, einem an den flüssigen Abzug angeschlossenen Umpumpkreislauf mit Pumpe und Wärmetauscher sowie mindestens einer Vorrichtung zur Aufgabe des umgepumpten Flüssigstroms auf die Packung oberhalb der Fangtasse.

In einer weiteren bevorzugten Ausführungsform des erfindungsgemäßen Verfahrens wird die Kondensation am Kopf der Destillationsvorrichtung so durchgeführt, dass ein Teilstrom vom Kopfaustrag in den Kondensator zurückgespült wird.

Um eine möglichst hohe Verfahrensausbeute bezüglich 1,3-Butadien trotz der nur teilweise erfolgten Umsetzung in Verfahrensschritt (1) zu erreichen, ist es bevorzugt, dass der azeotrop erhaltene Strom an 1,3-Butadien (Strom 2) in den Verfahrensschritt (1) zurückgeführt wird. Die Rückführung in Verfahrensschritt (1) kann gegebenenfalls auch nur teilweise erfolgen.

Der absolute Druck in Verfahrensschritt (2a) beträgt vorzugsweise 0,001 bis 100 bar, besonders bevorzugt 0,01 bis 10 bar, insbesondere 0,5 bis 5 bar. Die Destillation wird so durchgeführt, dass die Temperatur im Sumpf der Destillationsvorrichtung vorzugsweise 30 bis 140 °C, besonders bevorzugt 50 bis 150 °C, insbesondere 60 bis 120 °C, beträgt. Die Destillation wird so durchgeführt, dass die Kondensationstemperatur am Kopf der Destillationsvorrichtung vorzugsweise -50 bis 150 °C, besonders bevorzugt - 15 bis 60 °C, insbesondere 5 bis 45 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In Verfahrensschritt (2a) wird ein Azeotrop aus 1,3-Butadien und Cyanwasserstoff aus dem Hydrocyanierungsstrom abgetrennt. Der verbleibende Hydrocyanierungsstrom kann anschließend in einen Verfahrensschritt (2b) in eine weitere Destillationsvorrichtung überführt werden.

Der Verfahrensschritt (2b) des erfindungsgemäßen Verfahrens kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungsko-Ionnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer Apparatur durchgeführt werden.

In einer besonders bevorzugten Ausführungsform wird die Destillationsvorrichtung in Verfahrensschritt (2b) in Abtriebsfahrweise betrieben.

Die Destillationsvorrichtung ist vorzugsweise mit einer strukturierten Packung ausgestattet, die 2 bis 50, besonders bevorzugt 3 bis 40, insbesondere 4 bis 30, theoretische Trennstufen erzeugt.

In einer besonders bevorzugten Ausführungsform des erfindungsgemäßen Verfahrens werden die mindestens eine zur Destillationsvorrichtung von Verfahrensschritt (2b) gehörigen Verdampferstufen so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer, durch kurze Kontaktzeiten des Materials an der Verdampferoberfläche und möglichst geringe Temperaturen der Verdampferoberflächen erreicht wird.

Der absolute Druck in Verfahrensschritt (2b) beträgt vorzugsweise 0,001 bis 10 bar, besonders bevorzugt 0,010 bis 1 bar, insbesondere 0,020 bis 0,5 bar. Die Destillation wird so durchgeführt, dass die Temperatur im Sumpf der Destillationsvorrichtung vorzugsweise 30 bis 140 °C, besonders bevorzugt 40 bis 130°C, insbesondere 50 bis 120°C, beträgt. Die Destillation wird so durchgeführt, dass die Kondensationstemperatur am Kopf der Destillationsvorrichtung vorzugsweise -20 bis 140 °C, besonders bevorzugt -10 bis 80°C, insbesondere -5 bis 60 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

Um die Verfahrensausbeute an eingesetztem 1,3-Butadien in dem erfindungsgemäßen Verfahren zu erhöhen, ist es bevorzugt, dass das der in Verfahrensschritt (2b) über Kopf erhaltene Strom, enthaltend 1,3-Butadien und gegebenenfalls weiteren azeotrop mit 1,3-Butadien abdestillierten Cyanwasserstoff, in den Verfahrensschritt (1) zurückgeführt wird. Die Rückführung in Verfahrensschritt (1) kann gegebenenfalls auch nur teilweise erfolgen. Dabei kann der zurückgeführte Strom vor seiner Rückführung zusätzlich einer verfahrenstechnischen Aufarbeitung, beispielsweise einer Verdichtung auf einen höheren Druck, unterzogen werden.

In einer besonders bevorzugten Ausführungsform des erfindungsgemäßen Verfahrens wird der in Verfahrensschritt (2b) über Kopf erhaltene Strom über den Verfahrensschritt (2a) in den Verfahrensschritt (1) zurückgeführt.

In einer weiteren bevorzugten Ausführungsform des erfindungsgemäßen Verfahrens wird die Destillation bei mittleren Verweilzeiten der flüssigen Phase im Sumpfbereich der ein oder mehreren Destillationsvorrichtungen der Verfahrensschritt (2a) und (2b) von zusammen weniger als 10 Stunden, besonders bevorzugt weniger als 5 Stunden, insbesondere weniger als 1 Stunde, durchgeführt.

Die Destillation wird vorzugsweise so durchgeführt, dass die zu Verfahrensschritt (1) zurückgeführte Menge an 1,3-Butadien von 1 bis 1000 Gew.-ppm, besonders bevorzugt von 2 bis 500 Gew.-ppm, insbesondere bevorzugt 5 bis 200 Gew.-ppm, 2-Methyl-3-butennitril enthält. Dies gelingt beispielsweise durch geeignete Wahl des Rücklaufverhältnisses des kondensierten 1,3-Butadien-haltigen Kopfstromes des Schrittes (2) auf die geeignete Destillationsvorrichtung.

Weiterer Gegenstand der vorliegenden Erfindung ist die Verwendung von 1,3-Butadien zur azeotropen Destillation von Cyanwasserstoff.

### Beispiel:

Ein Reaktionsaustrag 1a aus der Hydrocyanierung von Butadien wird von 16 bar Reaktionsdruck über einen Flashtopf unmittelbar in eine Destillationskolonne mit Abtriebs- und Verstärkungsteil gespeist. Die Kolonne ist mit einer Packung MontzPak Typ B1-350 ausgestattet. Der Verstärkungsteil hat einen Innendurchmesser von 200 mm und eine Höhe von 3 m, der Abtriebsteil einen Innendurchmesser von 80 mm und eine Höhe von 2,5 m. Die Kolonne wird mit einem Zwangsumlaufverdampfer mit 1,1 m² Verdampferfläche betrieben, der durch eine Pumpe beschickt wird. Die Kondensation erfolgt über einen Rohrbündelwärmetauscher mit 2,2 m² Wärmeübertragungsfläche. Der Kondensator wird mit Sole gekühlt. Der Sumpfverdampfer wird mit Dampf beheizt.

Der Sumpf der Destillationskolonne ist als geteilter Sumpf ausgeführt. Auf der Rücklaufseite des geteilten Sumpfes wird ein Strom 1b eingespeist. Auf der Verdampferseite des Sumpfes wird über ein Thermoelement in einem Stutzen eine Temperatur von 90 °C gemessen. Dabei beträgt der Kopfdruck 2,02 bar und es stellt sich der Druckverlust über die Kolonne ein. Der Sumpfabzugsstrom 2s ist ebenfalls in Tabelle 1 aufgeführt.

Der Kondensatstrom hat eine Ablauftemperatur von 15 °C. Der Ablauf des Kondensators fließt durch eine Rohrleitung mit einem Innendurchmesser von 10 mm. An der Außenseite dieses Rohres ist zunächst ein kapazitiver Messaufnehmer der Fa. Endress und Hauser, Typ Multicap DC 16 montiert. Dieser Signalaufnehmer ist kalibriert auf Cyanwasserstoff-Konzentrationen in Butadien gemäß Tabelle 3. Bei Durchströmung der Leitung ergibt sich ein Messsignal von 27,0 % des maximalen analogen Ausgangssignals. Das Kondensat wird einem auf 0 °C temperierten Edelstahlbehälter aufgefangen und dort mit Frisch-Butadien (Strom 2b) gemischt. Aus diesem Behälter werden die Rücklaufmenge auf den Kolonnenkopf und die Abzugsmenge an Butadien zur Beschickung der Hydrocyanierung im Verhältnis 1 zu 7 abgezogen. Die Zusammensetzung des Abzugsstroms 2a ist ebenso in Tabelle 1 abzulesen.

Strom 1a enthält 0,03 Gew.-% Cyanwasserstoff, Strom 2s 0,41 Gew.-% und Strom 2a 0,03 Gew.-% (Bestimmung durch Absorption einer definierten Probe in Natronlauge und anschließender argentometrischer Cyanidtitration). Strom 2b stellt handelsübliches Butadien dar und ist frei von Cyanwasserstoff. Weitere Konzentrationen zu den Strömen in Tabelle 1 wurden über GC-Analytik (GC der Fa. Hewlett-Packard Typ 5890, Säule HP 50-1, interner Standard Benzonitril) ermittelt.

**Tabelle 1: Stromzusammensetzungen**

| | Strom Gew.-% | 1a Strom 1b Gew.-% | Strom 2s Gew.-% | Strom 2a Gew.-% | Strom 2b Gew.-% |
|---|---|---|---|---|---|
| Cyanwasserstoff (Titration) | 0,03 | 4,5 | 0,41 | 0,03 | 0,00 |
| 1,3-Butadien | 9,1 | 31,7 | 2,9 | 99,8 | 90,0 |
| 3-Pentennitril | 33,1 | 0,5 | 35,0 | 0,0 | 0,0 |
| 2-Methyl-3-butennitril | 29,6 | 4,9 | 31,8 | 0,0 | 0,0 |
| 2-Methylglutardinitril | 3,7 | 0,0 | 3,9 | 0,0 | 0,0 |
| 1-Buten | 2,7 | 7,3 | 0,7 | 0,0 | 5,5 |
| 2-Buten | 2,2 | 50,8 | 4,6 | 0,2 | 4,5 |
| Adipodinitril | 3,7 | 0,0 | 3,9 | 0,0 | 0,0 |

Die Restkonzentration zu 100 Gew.-% ergibt sich in den Strömen 1a und 2s dadurch, dass Katalysatorkomponenten im Reaktionsaustrag enthalten sind. Dies sind Ni(0)-Komplexe des Liganden der Formel A und freier Ligand der Formel A.

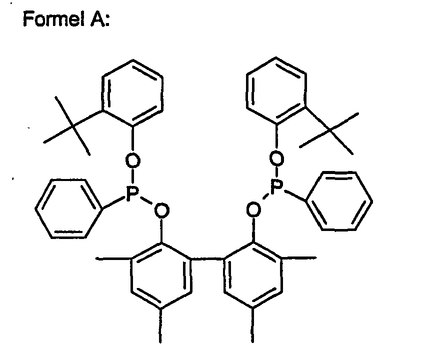

Die gemessenen Mengenströme sind in Tabelle 2 zusammengefasst.

**Tabelle 2: Strommengen**

| Stromnummer | Massenstrom |
|---|---|
| 1a | 60,4 kg/h |
| 1b | 5,0 kg/h |
| 2s | 57,1 kg/h |
| 2a | 29,6 kg/h |
| 2b | 21,2 kg/h |

**Tabelle 3: Kalibrierung kapazitive Sonde bei 15 °C**

| Cyanwasserstoff-Gehalt in 1,3-Butadien | Ausgangssignal bzgl. maximaler Signalausgang |
|---|---|
| 0,0 Gew.-% | 25,3 % |
| 0,8 Gew.-% | 34,5 % |

## Patentansprüche

1. Verfahren zur Verringerung des Gehaltes an Cyanwasserstoff in Pentennitrilen und Cyanwasserstoff enthaltenden Mischungen, **dadurch gekennzeichnet, dass** die Verringerung des Gehaltes an Cyanwasserstoff durch eine Abtrennung von Cyanwasserstoff mittels einer azeotropen Destillation mit 1,3-Butadien erfolgt.

2. Verfahren nach Anspruch 1, **gekennzeichnet durch** die folgenden Verfahrensschritte:
(1) Hydrocyanierung von 1,3-Butadien **durch** dessen Umsetzung mit Cyanwasserstoff in Gegenwart mindestens eines Hydrocyanierungskatalysators unter Erhalt eines Hydrocyanierungsstromes, der 3-Pentennitril, 2-Methyl-3-butennitril, Cyanwasserstoff, 1,3-Butadien und den mindestens einen Hydrocyanierungskatalysator enthält,
(2) Abtrennung eines Gemisches aus Cyanwasserstoff und 1,3-Butadien, das ein Azeotrop bildet, aus dem Hydrocyanierungsstrom **durch** Destillation.

3. Verfahren nach Anspruch 2, **dadurch gekennzeichnet, dass** das in Verfahrensschritt (2) verwendete 1,3-Butadien aus dem Verfahrensschritt (1) stammt.

4. Verfahren nach Anspruch 2, **dadurch gekennzeichnet, dass** das in Verfahrensschritt (2) verwendete 1,3-Butadien dem Hydrocyanierungsstrom vor der azeotropen Destillation zugesetzt wird.

5. Verfahren nach Anspruch 2, **dadurch gekennzeichnet, dass** das in Verfahrensschritt (2) verwendete 1,3-Butadien zum Teil aus dem Verfahrensschritt (1) stammt und zum Teil dem Hydrocyanierungsstrom vor der azeotropen Destillation zugesetzt wird.

6. Verfahren nach Anspruch 2, **dadurch gekennzeichnet, dass** das gesamte 1,3-Butadien, das in Verfahrensschritt (1) zugeführt wird, dem Strom, der aus dem Verfahrensschritt (1) zum Verfahrensschritt (2) geführt wird, zugesetzt wird und von Verfahrensschritt (2) in Verfahrensschritt (1) zurückgeführt wird.

7. Verfahren nach einem der Ansprüche 2 bis 6, **dadurch gekennzeichnet, dass** in Verfahrensschritt (1) ein Nickel(0)-Katalysator als Hydrocyanierungskatalysator verwendet wird.

8. Verfahren nach einem der Ansprüche 1 bis 7, **dadurch gekennzeichnet, dass** die Pentennitril enthaltende Mischung oder der in Verfahrensschritt (1) erhaltene Hydrocyanierungsstrom mindestens einen der folgenden Gehalte aufweist:
- 10 bis 99 Gew.-% Pentennitrile, umfassend trans-3-Pentennitril, 2-Methyl-3-butennitril sowie weitere Pentennitrilisomere, wobei je nach verwendetem Katalysatorsystem und Reaktionsbedingungen das Verhältnis von 2-Methyl-3-butennitril zu trans-3-Pentennitril 0,1 : 1 bis 5 : 1 betragen kann;
- 0 bis 60 Gew.-% 1,3-Butadien;
- 0,01 bis 30 Gew.-% Katalysatorkomponenten einschließlich Katalysatorabtropfprodukte;
- 0 bis 10 Gew.-% Cyanwasserstoff.

9. Verfahren nach einem der Ansprüche 1 bis 8, **dadurch gekennzeichnet, dass** die azeotrope Destillation von 1,3-Butadien und Cyanwasserstoff bei einer Temperatur im Sumpf einer geeigneten Destillationsvorrichtung von 30 bis 250 °C und bei einer Temperatur bei der Kondensation in einer geeigneten Destillationsvorrichtung von -50 bis 150 °C und/oder einem Druck von 0,001 bis 100 bar erfolgt.

10. Verwendung von 1,3-Butadien zur azeotropen Destillation von Cyanwasserstoff.

## Claims

1. A process for reducing the content of hydrogen cyanide in mixtures comprising pentenenitriles and hydrogen cyanide, which comprises reducing the content of hydrogen cyanide by a removal of hydrogen cyanide by means of an azeotropic distillation with 1,3-butadiene.

2. The process according to claim 1, **characterized by** the following process steps:
(1) hydrocyanating 1,3-butadiene by its reaction with hydrogen cyanide in the presence of at least one hydrocyanation catalyst to obtain a hydrocyanation stream which comprises 3-pentenenitrile, 2-methyl-3-butenenitrile, hydrogen cyanide, 1,3-butadiene and the at least one hydrocyanation catalyst,
(2) removing a mixture of hydrogen cyanide and 1,3-butadiene which forms an azeotrope from the hydrocyanation stream by distillation.

3. The process according to claim 2, wherein the 1,3-butadiene used in process step (2) stems from process step (1).

4. The process according to claim 2, wherein the 1,3-butadiene used in process step (2) is added to the hydrocyanation stream before the azeotropic distillation.

5. The process according to claim 2, wherein the 1,3-butadiene used in process step (2) partly stems from process step (1) and is partly added to the hydrocyanation stream before the azeotropic distillation.

6. The process according to claim 2, wherein all of the 1,3-butadiene which is added in process step (1) is added to the stream which is conducted from process step (1) to process step (2), and is recycled from process step (2) into process step (1).

7. The process according to any of claims 2 to 6, wherein the hydrocyanation catalyst used in process step (1) is a nickel(0) catalyst.

8. The process according to any of claims 1 to 7, wherein the pentenenitrile-comprising mixture or the hydrocyanation stream obtained in process step (1) has at least one of the following contents:
- from 10 to 99% by weight of pentenenitriles comprising trans-3-pentenenitrile, 2-methyl-3-butenenitrile and also further pentenenitrile isomers, the ratio of 2-methyl-3-butenenitrile to trans-3-pentenenitrile being from 0.1:1 to 5:1 depending on the catalyst system and reaction conditions used;
- from 0 to 60% by weight of 1,3-butadiene;
- from 0.01 to 30% by weight of catalyst components including catalyst dripping products;
- from 0 to 10% by weight of hydrogen cyanide.

9. The process according to any of claims 1 to 8, wherein the azeotropic distillation of 1,3-butadiene and hydrogen cyanide is effected at a temperature in the bottom of a suitable distillation apparatus of from 30 to 250°C and at a temperature in the condensation in a suitable distillation apparatus of from -50 to 150°C and/or a pressure of from 0.001 to 100 bar.

10. The use of 1,3-butadiene for azeotropically distilling hydrogen cyanide.

## Revendications

1. Procédé de réduction de la teneur en cyanure d'hydrogène dans des mélanges contenant des pentène-nitriles et du cyanure d'hydrogène, **caractérisé en ce que** la réduction de la teneur en cyanure d'hydrogène a lieu par une séparation de cyanure d'hydrogène au moyen d'une distillation azéotropique avec du 1,3-butadiène.

2. Procédé selon la revendication 1, **caractérisé par** les étapes de procédé suivantes :
(1) l'hydrocyanation de 1,3-butadiène par sa réaction avec du cyanure d'hydrogène en présence d'au moins un catalyseur d'hydrocyanation pour obtenir un courant d'hydrocyanation, qui contient du 3-pentène-nitrile, du 2-méthyl-3-butène-nitrile, du cyanure d'hydrogène, du 1,3-butadiène et le ou les catalyseurs d'hydrocyanation,
(2) la séparation d'un mélange de cyanure d'hydrogène et de 1,3-butadiène, qui forme un azéotrope, du courant d'hydrocyanation par distillation.

3. Procédé selon la revendication 2, **caractérisé en ce que** le 1,3-butadiène utilisé à l'étape de procédé (2) est issu de l'étape de procédé (1).

4. Procédé selon la revendication 2, **caractérisé en ce que** le 1,3-butadiène utilisé à l'étape de procédé (2) est ajouté au courant d'hydrocyanation avant la distillation azéotropique.

5. Procédé selon la revendication 2, **caractérisé en ce que** le 1,3-butadiène utilisé à l'étape de procédé (2) est issu en partie de l'étape de procédé (1) et est ajouté en partie au courant d'hydrocyanation avant la distillation azéotropique.

6. Procédé selon la revendication 2, **caractérisé en ce que** la totalité du 1,3-butadiène introduit dans l'étape de procédé (1) est ajouté au courant qui est conduit dans l'étape de procédé (2) à partir de l'étape de procédé (1), et recyclé de l'étape de procédé (2) dans l'étape de procédé (1).

7. Procédé selon l'une quelconque des revendications 2 à 6, **caractérisé en ce qu'**un catalyseur de nickel (0) est utilisé en tant que catalyseur d'hydrocyanation à l'étape de procédé (1).

8. Procédé selon l'une quelconque des revendications 1 à 7, **caractérisé en ce que** le mélange contenant du pentène-nitrile ou le courant d'hydrocyanation obtenu à l'étape de procédé (1) présente au moins une des teneurs suivantes :
- 10 à 99 % en poids de pentène-nitriles, comprenant du trans-3-pentène-nitrile, du 2-méthyl-3-butène-nitrile et d'autres isomères de pentène-nitrile, le rapport entre le 2-méthyl-3-butène-nitrile et le trans-3-pentène-nitrile pouvant être de 0,1:1 à 5:1 en fonction du système catalytique et des conditions de réaction utilisés ;
- 0 à 60 % en poids de 1,3-butadiène ;
- 0,01 à 30 % en poids de composants catalytiques, y compris les produits d'égouttement du catalyseur ;
- 0 à 10 % en poids de cyanure d'hydrogène.

9. Procédé selon l'une quelconque des revendications 1 à 8, **caractérisé en ce que** la distillation azéotropique de 1,3-butadiène et de cyanure d'hydrogène a lieu à une température dans le fond d'un dispositif de distillation approprié de 30 à 250 °C et à une température lors de la condensation dans un dispositif de distillation approprié de -50 à 150 °C et/ou à une pression de 0,001 à 100 bar.

10. Utilisation de 1,3-butadiène pour la distillation azéotropique de cyanure d'hydrogène.
